# EUROPÄISCHE PATENTSCHRIFT

(11) **EP 0 388 944 B1**
(45) Veröffentlichungstag und Bekanntmachung des Hinweises auf die Patenterteilung: **02.02.1994**
(21) Anmeldenummer: 90105402.3
(22) Anmeldetag: 22.03.1990
(51) Int. Cl.: A61N 5/06

(54) **Bräunungsvorrichtung**
Tanning device
Dispositif de bronzage

(30) Priorität: 23.03.1989 DE 8903687 U; 01.04.1989 DE 8903975 U
(43) Veröffentlichungstag der Anmeldung: 26.09.1990
(73) Patentinhaber: Danhauser, Thomas, D-93128 Regenstauf (DE)
(72) Erfinder: Danhauser, Thomas, D-93128 Regenstauf (DE)
(74) Vertreter: Wasmeier, Alfons, Dipl.-Ing.

(56) Entgegenhaltungen:
- DE-A- 3 229 690
- DE-A- 3 533 789
- DE-A- 3 632 927
- DE-A- 3 708 872

## Beschreibung

Die Erfindung bezieht sich auf eine Bräunungsvorrichtung mit den Merkmalen des Oberbegriffes des Anspruches 1.

Herkömmliche Bräunungsvorrichtungen bestehen aus einer Liege mit lichtdurchlässiger Auflage, Reflektorlampen, die über die Oberfläche verteilt sind und die das Licht der Lampen gegen die auf der Liege ruhende Person richten, einem Schwenkhimmel mit Reflektorlampen, der als Deckel mit Handbetrieb oder Motorbetrieb gegen die Liege einstellbar ist, einem Lüftungssystem, das die durch die Lampen erzeugte Wärme abführt, und einem Steuergerät, mit dem die Zustellung des Schwenkhimmels, die Schaltung der Lampen usw. gesteuert wird.

Derartige, als Liegen ausgebildete Anlagen sind relativ aufwendig, haben große Abmessungen und sind damit teuer. Bei beengten Platzverhältnissen kommt deshalb in der Regel die Aufstellung einer als Liege ausgebildeten Anlage nicht in Frage. Des weiteren haben Liegen den Nachteil, daß aufgrund der in Längsachse der Liege verlaufenden Lampenröhren, die sich vom Kopfende zum Fußende der Liege erstrecken, der volle Strahlenfluß der Lampen an den Lampenenden nicht aufrechterhalten werden kann, so daß zusätzliche Gesichtsbräuner benötigt werden. Weiterhin hat sich gezeigt, daß bei liegender Körperstellung, insbesondere bei Benutzern mit Kreislaufschwierigkeiten und Lendenwirbelbeschwerden, erhebliche Probleme auftreten können. Die große Länge der bei Liegen benötigten Lampen (mindestens 2,10 m) stellt auch einen relativ großen Aufwand bei der Herstellung, beim Transport und beim Handling solcher Lampen dar.

Ein Liegegestell in kürzerer Bauweise zeigt der Vorschlag nach der DE-A-36 32 927. Diese Liegegestell ist in Form eines Liegestuhles ausgebildet, der aus einer Körperauflageplatte als Unterteil besteht, die drei stumpfwinklig zueinander verlaufende Teilabschnitte umfaßt, deren mittlerer Abschnitt die Sitzfläche, deren unterer Abschnitt den Fußteil und deren oberer Abschnitt den Rücken-und Kopfteil bildet, wobei Fuß- und Rückenteil parallel zueinander angeordnet sind. Dem Unterteil ist ein in der Form entsprechendes Oberteil zugeordnet, derart, daß Oberteil und Unterteil an allen Stellen über die Längs- und Querachse den gleichen Abstand haben. Fuß- und Kopfteil des Unterteiles sind im Winkel verstellbar angeordnet. Weiterhin ist das Oberteil um eine horizontale Achse schwenkbar an einem Tragständer gelagert und gegenüber dem Unterteil abstandsverstellbar. Das Unterteil weist auf der Liegefläche eine Vielzahl von einzelnen versenkten, gewölbten Reflektoren, die sowohl in Längs- als in Querrichtung durch die Liegefläche unterbrochen sind, und innerhalb dieser Reflektoren kurze Bestrahlungslampen auf, so daß die Strahlungsabgabe nur über einen beschränkten Teil der Breite und Länge des Liegegestells erfolgt.

Aufgabe der Erfindung ist es, eine Bräunungsvorrichtung zu schaffen, die erheblich weniger Platz beansprucht als eine Liege, bei der der Benutzer während des Bräunens eine bequeme sitzende Position einnehmen kann, ohne daß der Bräunungseffekt nachteilig beeinflußt wird, die einen besonders bequemen Ein- und Ausstieg ermöglicht, und die wesentlich wirtschaftlicher und mit geringerem Stromverbrauch als herkömmliche Bräunungsvorrichtungen betrieben werden können.

Dies wird gemäß der Erfindung mit den Merkmalen des Kennzeichens des Anspruches 1 erreicht. Weitere Ausgestaltungen der Erfindung sind Gegenstand der Unteransprüche.

Die erfindungsgemäße Bräunungsvorrichtung ist ein Bräunungssessel mit einer unteren Einheit (Unterteil) bestehend aus einer horizontalen Sitzfläche, einer schrägen, im Winkel a von zwischen 30° und 60° geneigten Rückenlehne und einer im Winkel β von zwischen 20° und 70° geneigten Fußstütze, sowie einer oberen Einheit (Oberteil), die aus etwa parallelen, entsprechenden Abschnitten besteht und die auf die untere Einheit zu und von ihr weg verstellbar ist. Die einzelnen Abschnitte der unteren und der oberen Einheit verlaufen in einem weitgehend konstanten Abstand voneinander und können in der Längsachse des Sessels, zumindest im Kopf-, Rücken- und Beckenbereich zur Anpassung an die Form des menschlichen Körpers muldenförmig ausgebildet sein.

Die untere und die obere Einheit sind jeweils unabhängig voneinander belüftet; die Belüftungs- und Kühlluft wird von unten an der vorderen Stirnseite angesaugt und oben an der hinteren Stirnseite als Abluft abgegeben. Die im unteren und oberen Teil angeordneten Lampen, vorzugsweise UVA-Lampen, sind in engem Abstand voneinander vorgesehen, um eine große Packungsdichte und damit einen hohen Bräunungseffekt zu erreichen.

Wenn die Neigung der Rückenlehne des Bräunungssessels relativ zur Sitzfläche verstellbar ausgebildet wird, ist es erforderlich, an der Schwenkstelle Abstand und Anordnung der Lampen so zu gestalten, daß bei einer Verschwenkung die benachbarten Lampen der Rückenlehne und der Sitzfläche nicht aufeinandertreffen und ein Kippen der Rückenlehne nicht behindern bzw. durch ein solches Kippen die Lampen nicht beschädigt werden.

Bei einer bevorzugten Ausführungsform der Erfindung verlaufen Oberteil und Unterteil nicht exakt parallel zueinander, sondern haben einen in etwa der Form des Körpers des Bräunenden angepaßten Abstand zueinander, und zwar im Brust- und Beckenbereich einen etwas größeren und im Bereich vom Oberschenkel abwärts und insbes. im Kniebereich einen etwas geringeren Abstand als dem mittleren Abstand entspricht, so daß bei gleicher Packungsdichte der Lampen eine gleichmäßigere Bräunung des gesamten Körpers erreicht wird. Wahlweise kann entsprechend die Packungsdichte oder die Leuchtstärke der Lampen an die unterschiedliche Körperform angepaßt werden, was jedoch insgesamt eine geringere Effektivität der Anlage ergeben würde. Der unterschiedliche Abstand ist vorzugsweise so bemessen, daß die beiden die Rückenlehne bildenden Abschnitte des Oberteiles und des Unterteiles sich nach unten in einem Winkel von ca. 2 - 6° erweitern, die beiden die Sitzfläche bildenden Abschnitte sich zum Fußteil in einem Winkel von ca. 5 - 10° verengen, und die beiden die Fußstütze bildenden Abschnitte etwa parallel oder sich nach unten in einem Winkel von ca. 2 - 6° verengend ausgebildet sind.

Die unterhalb der Sitzfläche angeordnete Steuereinheit, die den Sockel des Sessels darstellt, nimmt den Antriebsmotor für das Verschwenken bzw. Heben und Senken des oberen Teiles der Anlage, die Steuergeräte für das Schalten der Lampen, die Schaltgeräte für die Belüftung usw. auf, so daß alle Steuervorgänge vom Benutzer in der Sitzstellung vorgenommen werden können. Dies gilt auch, wenn die Antriebsbewegungen für das Schwenken bzw. Heben und Senken des oberen Teiles von Hand vorgenommen werden, wobei die Bewegung durch eine Übersetzung erleichtert wird.

Nach einer speziellen Ausführungsform der Erfindung ruht der Sockel auf einer Grundplatte bzw. einem Grundsockel, der einen weiteren Antrieb enthält, welcher die gesamte Bräunungsanlage mit Sockel z.B. in eine leicht wippende Bewegung um eine Achse quer zur Längsachse der Bräunungsvorrichtung versetzt (leichte Schaukelbewegung). Der Wippenausschlag ist zwischen einem Nullwert und einem maximalen Wert von ca. 10-15 cm einstellbar ausgebildet. Diese Wipp- bzw. Schwenkbewegung kann durch Programm gesteuert werden, oder sie kann kontinuierlich oder diskontinuierlich durch Schalten des Antriebs erreicht werden. Diese Bewegung des gesamten Bräunungssessels hat den Zweck, die Auflage der Wirbelsäule und den auf den Körper wirkenden Druck zu verändern und dadurch die Wirbelsäule zu entlasten. Zweckmäßigerweise erfolgt dieser Wipp- bzw. Schwenkvorgang selbsttätig und kontinuierlich, er ist jedoch so ausgelegt, daß er von dem Benutzer auch direkt beeinflußt und an- und abgeschaltet werden kann.

Die Lampen sind bei dem erfindungsgemäßen Sessel parallel zur Querachse des Sessels angeordnet und kleiner als die Breite des Sessels gehalten. Dadurch ist es bei verhältnismäßig hoher Packungsdichte der Lampen möglich, mit einer kleineren Gesamtleistung auszukommen, die bei einer speziellen Ausführungsform bei 3200 Watt und damit unter dem Wert liegt, bei dem ein Starkstromanschluß erforderlich ist, so daß der Bräunungssessel nach der Erfindung für einen Anschluß an eine Steckdose mit 220 V zulässig ist. Bei herkömmlichen Liegen, die einen Anschlußwert von ca. 3600 Watt oder darüber haben, ist hingegen ein Starkstromanschluß notwendig.

Der untere Teil und/oder der obere Teil des erfindungsgemäßen Bräunungssessels weist bei einer speziellen Ausführungsform der Erfindung einen Seitenflügel auf, der gegenüber dem Hauptteil im Winkel angeordnet ist, mit UVA-Lampen ausgerüstet ist und Strahlung seitlich gegen den Körper des Bräunenden richtet. Der Neigungswinkel des Seitenflügels ist verhältnismäßig groß, damit eine seitliche Bestrahlung erreicht wird, etwa zwischen 30 und 90°. Dabei ist der Seitenflügel z.B. im oberen Teil auf der einen und im unteren Teil auf der anderen Seite so ausgebildet, daß der Bräunungssessel teilweise geschlossen und damit der Bräunungseffekt im seitlichen Bereich erheblich verbessert wird. Ein bevorzugter Wert des Neigungswinkels dieser Seitenflügel liegt zwischen 45 und 60°.

Zur Verbesserung der Lüftung, der Führung des Kühlluftstromes und der Kühlung sowohl der Liegefläche selbst als der UVA-Lampen sind zumindest im unteren Teil der Bräunungsvorrichtung quer zu deren Längsachse und parallel zur Längsachse der einzelnen Lampen verlaufende, durchgehende Stege bzw. Trennwände vorgesehen, die Lochungen für das Durchblasen von Kühlluft aufweisen; die Stege bzw. Trennwände sind zur Aufnahme der Auflageplatte ausgebildet. Durch die Stege mit Lochungen wird eine Verwirbelung und damit eine ungleichmäßige Strömungsgeschwindigkeit des Kühlluftstromes erreicht, die den Wärmeübergang bzw. die Wärmeaufnahme im Kühlluftstrom wesentlich verbessert und damit die Effektivität der Kühlung der Auflageplatte und der UVA-Lampen erhöht, so daß letztlich die Leistung des Lüftungs- und Kühlsystems geringer ausgelegt werden kann. Die Lochungen in den Stegen bzw. Trennwänden zwischen Auflageplatte und Boden des unteren Teiles der Bräunungsvorrichtung sind von kreisförmiger, langlochförmiger oder ähnlicher Gestalt und vorzugsweise in der oberen Hälfte der Stege nahe der Auflageplatte vorgesehen, so daß der Luftstrom im unteren Teil der Stege auf eine geschlossene Wand auftrifft, dabei eine erhebliche Verwirbelung vor dem Eintritt in die jeweiligen Lochungen erfährt und im oberen Teil der Stege teilweise auf die die Lochungen begrenzende Wandfläche auftrifft und teilweise durch die Lochungen mit erhöhter Geschwindigkeit strömt. In den durch die Stege gebildeten und durch die Lochungen miteinander verbundenen Kammern sind die UVA-Lampen untergebracht, so daß der Luftstrom die Lampen voll umströmt und kühlt, gleichzeitig aber auch durch den Durchtritt im oberen Bereich der Stege eine besonders effektive Kühlung der Unterseite der Auflagefläche ergibt.

Nachstehend wird die Erfindung in Verbindung mit der Zeichnung anhand zweier Ausführungsbeispiele erläutert. Es zeigen:
- Fig. 1: eine schematische Seitenansicht eines Bräunungssessels mit schwenkbarem oberem Teil in seitlicher Ansicht,
- Fig. 2: den Bräunungssessel nach Fig. 1 in einer Ansicht von vorne,
- Fig. 3: eine Seitenansicht eines Bräunungssessels mit einer seitlichen Liftvorrichtung zum Heben und Senken des oberen Teiles,
- Fig. 4: eine Ansicht des Bräunungssessels nach Fig. 3 von vorne,
- Fig. 5: eine perspektivische Ansicht des Bräunungssessels nach Fig. 3 bzw. Fig. 4,
- Fig. 6: eine schematische Seitenansicht eines Bräunungssessels mit unterschiedlichem Abstand zwischen oberem und unterem Teil,
- Fig. 7: eine schematische Darstellung einer Ausführungsform einer Wippenanordnung, mit der der Bräunungssessel kontinuierlich während des Betriebes verschwenkbar ist,
- Fig. 8: eine schematische Darstellung der Seitenflügel im Schnitt, und
- Fig. 9: eine schematische Darstellung einer Ausführungsform einer Belüftungsvorrichtung im Schnitt.

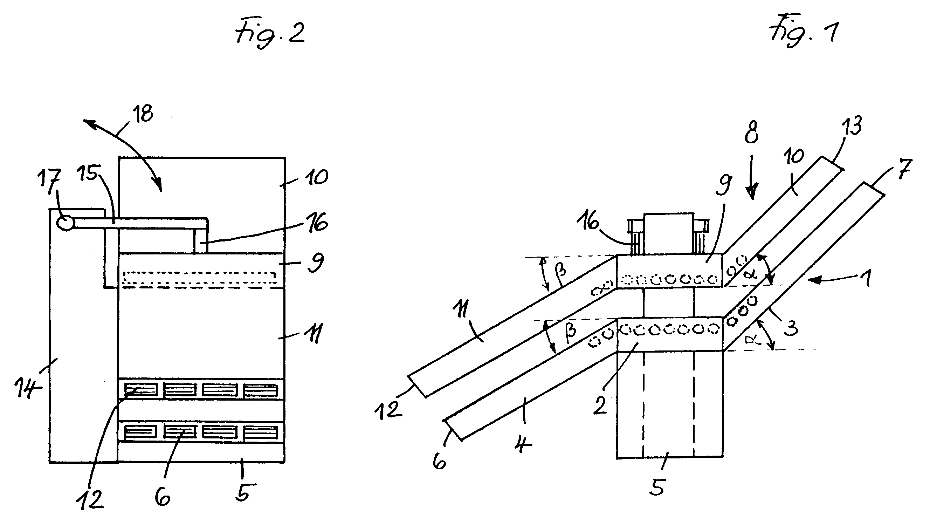

Der untere Teil 1 des Bräunungssessels nach Fig. 1 bzw. 2 besteht aus der Sitzfläche 2, der Rückenlehne 3 und der Fußstütze 4, und ruht auf einem Sockel 5, der die (nicht dargestellte) Steuer- und Antriebseinheit aufnimmt. Am unteren Ende der Fußstütze 4 sind mit 6 Belüftungsöffnungen angedeutet, entsprechende Abluftöffnungen sind mit 7 am oberen Ende der Rückenlehne vorgesehen. 8 bezeichnet den oberen Teil des Bräunungssessels. Dieser Teil 8 ist in der Formgebung der Formgebung des unteren Teiles 1 angepaßt und weist einen der Sitzfläche 2 angepaßten und zugewandten horizontalen Teil 9, einen der Rückenlehne 3 angepaßten und zugewandten geneigten Abschnitt 10 sowie einen der Fußstütze 4 angepaßten und zugewandten Teil 11 auf, der Belüftungsöffnungen 12 analog den Belüftungsöffnungen 6 besitzt. Im oberen Abschnitt 10 sind Abluftöffnungen 13 entsprechend den Abluftöffnungen 7 vorgesehen.

Am Sockel 5 ist ein nach oben verlängerter seitlicher Träger 14 befestigt, mit dem ein horizontal und quer über den oberen Teil 8 verlaufender Tragarm 15 verbunden ist, der an seinem vom Träger 14 abgewandten Ende eine Verbindung 16 zum Abschnitt 9 des oberen Teiles 8 besitzt. Der Tragarm 15 ist dabei im Träger 14 schwenkbar gelagert, derart, daß - wie in der Darstellung nach Fig. 2 gezeigt - der Tragarm 15 mit dem oberen Teil 8 des Bräunungssessels im Gegenuhrzeigersinn um einen Schwenkpunkt 17 verschwenkbar ist (Pfeil 18).

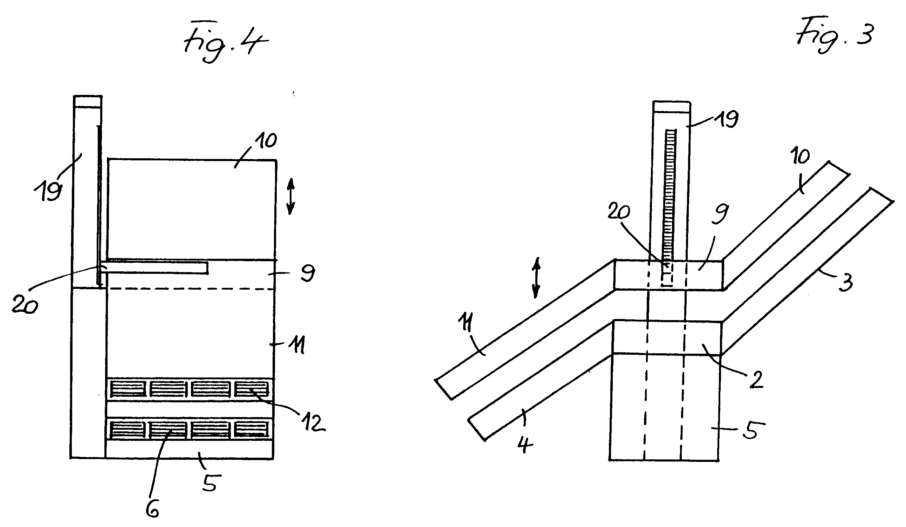

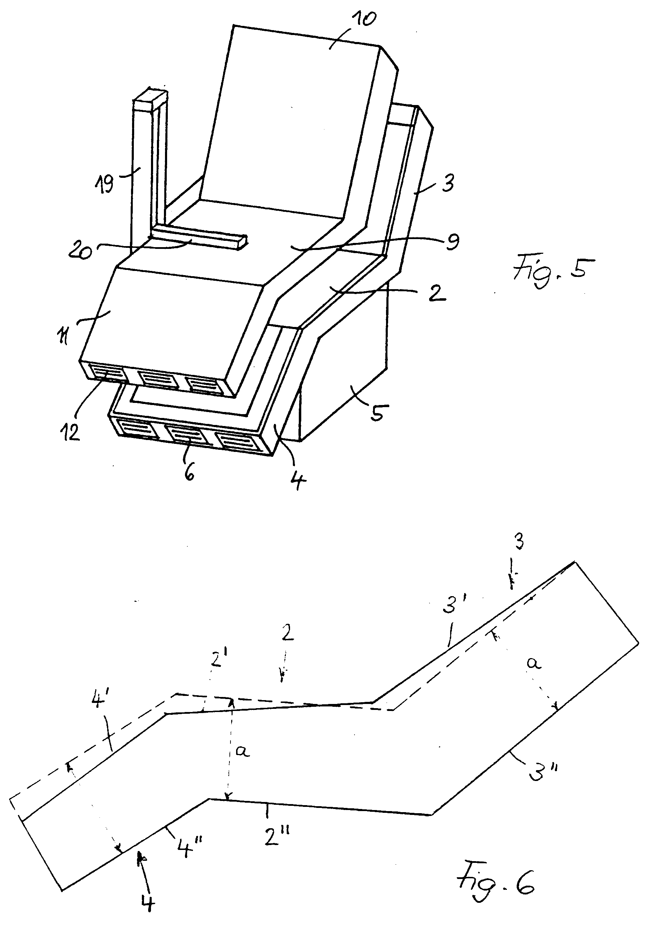

Die Ausgestaltung und Anordnung des Bräunungssessels nach den Fig. 3, 4 und 5 ist ähnlich der nach Fig. 1 und 2, jedoch ist der obere Teil 8 des Bräunungssessels heb- und senkbar (anstatt schwenkbar). Hierzu ist am Sockel 5 ein verlängerter Träger 19 vorgesehen, der einen Tragarm 20 aufweist, welcher mit dem Abschnitt 9 des oberen Teiles 8 des Bräunungssessels fest verbunden ist, und welcher im Träger 19, z.B. über ein Ritzel-Zahnstangen-Getriebe auf und ab bewegbar ist. Diese Bewegung erfolgt so, daß der obere Teil 9 bei seiner Bewegung parallel zum Teil 8 bewegt wird. Die Steuereinheit im Sockel 5 kann in sitzender Position betätigt werden. Die Vorrichtung, mit der die Hubbewegung des oberen Teiles 8 durchgeführt wird, ist für die Erfindung nicht ausschlaggebend, d.h. daß eine beliebige, geeignete Liftvorrichtung bekannter Art verwendet werden kann.

Die schematische Darstellung nach den Fig. 1 und 2 zeigt einen einzigen Tragarm 15, der den oberen Teil 8 in Pfeilrichtung 18 um die Schwenkstelle 17 verschwenkt. Vom Träger 14 können jedoch auch zwei Tragarme 15 ausgehen, deren einer mit dem Abschnitt 10 und deren anderer mit dem Abschnitt 11 verbunden ist, und zwar jeder in einem relativ geringen Abstand vom jeweiligen Ende des Bereiches 9. Auch bei der Ausführungsform nach den Fig. 3 - 5 können anstelle eines Tragarmes 20 zwei im Abstand voneinander angeordnete Tragarme vorgesehen sein, um die Last auf zwei Stellen zu verteilen.

In der schematischen Darstellung nach Fig. 6 ist gestrichelt der Verlauf der Abschnitte 9, 10, 11 angedeutet, wobei Oberteil und Unterteil jeweils in gleichem Abstand voneinander angeordnet sind. Voll ausgezogen ist eine abgeänderte Ausführungsform gezeigt, bei der der Abstand des Oberteiles vom Unterteil in allen drei Abschnitten 9, 10 und 11 einen etwas unterschiedlichen Wert hat, weil der Abstand zwischen Oberteil und Unterteil bis zu einem gewissem Grad dem Verlauf der Körperform des Bräunenden angepaßt ist; im oberen Bereich 10 verlaufen Oberteil und Unterteil nach unten etwas auseinander, z.B. in einem Winkel zwischen 2 und 6°, im mittleren Abschnitt verringern Oberteil und Unterteil ihren Abstand voneinander in Richtung Fußstütze etwas, z.B. 5 - 10°, und im unteren Abschnitt erstrecken sich Oberteil und Unterteil nach unten im wesentlichen parallel zueinander oder in geringem Maße konisch (ca. 2 - 6°) nach unten. Die Formgebung des Unterteiles sowie des Oberteiles und der unterschiedliche Abstand zwischen beiden kann dabei noch mehr der Körperform angepaßt werden, falls dies erwünscht ist, indem die Neigung der einzelnen Abschnitte des Oberteiles gegenüber denen des Unterteiles in ihrer Neigung der Kontur der Oberseite und der Unterseite des Körpers entsprechend ausgebildet ist und die Übergänge von einem Abschnitt zum nächsten gerundet sind.

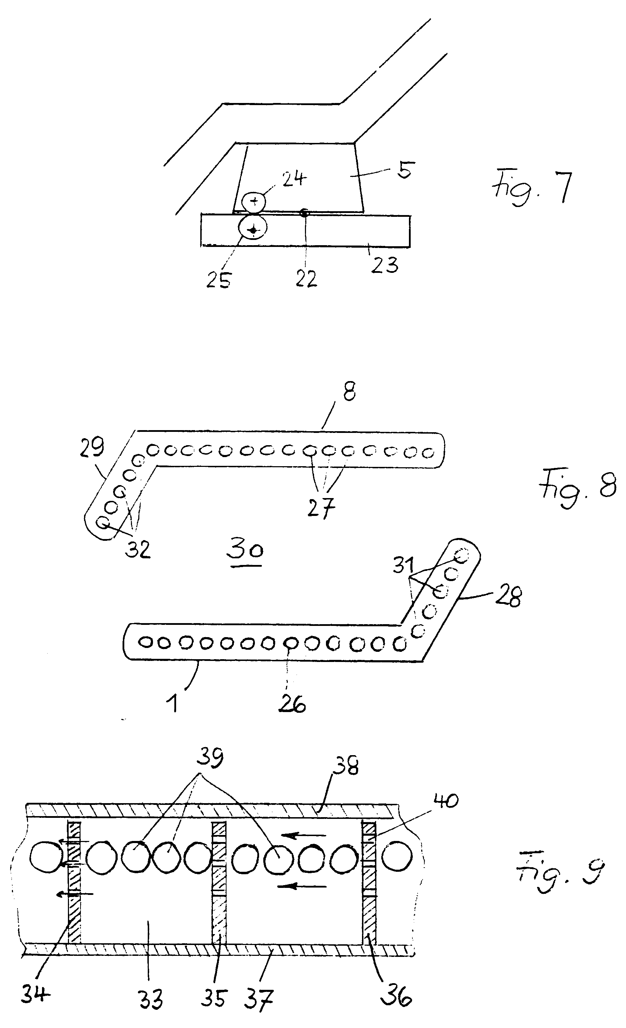

Fig. 7 zeigt schematisch eine Ausführungsform einer Wippenanordnung dargestellt, mit deren Hilfe der gesamte Bräunungssessel um eine Querachse 22 schwenkbar bzw. kippbar ist. Zu diesem Zweck ist der die Querachse 22 aufnehmende Sockel 5 auf einer Grundplatte bzw. einem Grundsockel 23 befestigt, und am Sockel 5 ist im unteren Bereich eine Rad-, Rollen-, Walzen- oder dergl. Anordnung 24 vorgesehen, mit der eine exzentrisch in der Grundplatte 23 gelagerte Rad-, Rollen-, Walzen- oder dergl. Anordnung 25 in Eingriff steht, die motorisch angetrieben ist und die die Anordnung 24 mit dem Sockel 5 und dem gesamten Bräunungssessel kontinuierlich um die Achse 22 schwenkt. Das Ausmaß der Schwenkbewegung am Bräunungssessel entspricht dabei dem Exzenterhub; ggf. kann eine Übersetzung eingeschaltet werden. Anstelle eines derartigen Exzenterantriebes 24, 25 kann jedoch auch ein beliebiger anderer Antrieb verwendet werden, z.B. eine Kolbensteuerung oder eine Zahnstangensteuerung, mit der der Sockel 5 mit Bräunungssessel angehoben und abgesenkt und dabei um die Schwenkachse 22 verschwenkt wird.

In Fig. 8 ist in schematischer Schnittansicht der obere Teil 8 und der untere Teil 1 mit den UVA-Lampen 26 und 27 und dem am unteren Teil 1 auf der rechten Seite angeordneten Seitenflügel 28 sowie im oberen Teil 8 auf der linken Seite angeordneten Seitenflügel 29 dargestellt. Die beiden Seitenflügel sind durchgehend parallel zur Längsachse des Bräunungssessels angeordnet und umschließen einen Raum 30, in dem der Bräunende sitzt, teilweise, wenn der obere Teil 8 gegen den unteren Teil 1 in die in Fig. 8 dargestellte Position abgesenkt und der Bräunungsstuhl geschlossen ist. Damit wird erreicht, daß eine Bräunung der Seitenbereiche des Körpers über die Lampen 31 und 32 erzielt wird.

Im Unterteil des Bräunungssessels (und wahlweise entsprechend auch im Oberteil) sind Lufteinlaß- und Luftauslaßschlitze in Verbindung mit einem Ventilator (in Fig. 9 nicht dargestellt) vorgesehen, so daß ein Luftführungskanal 33 gebildet wird, in dem eine durch Pfeile angedeutete Luftströmung erfolgt. Quer zur Längsachse des Unterteiles sind von der einen zur anderen Seite des Unterteiles Stege 34, 35, 36 in Form von den Kanal 33 von einer Seite zur anderen durchsetzenden Trennwänden angeordnet, die mit dem Boden 37 des Unterteiles bzw. mit dem darunter befindlichen Sockel fest verbunden sind. Auf den Stegen 34, 35, 36 ruht eine Auflageplatte 38, auf der der Bräunende Platz nimmt. Die Auflageplatte 38 stellt die Oberseite des Unterteiles dar. Die Stege 34, 35, 36 bilden untereinander und zusammen mit den Seitenwänden des Unterteiles einzelne Kammern aus, die nach oben durch die Auflageplatte 38 und nach unten durch die Bodenwand 37 begrenzt sind. In diesen Kammern sind die UVA-Lampen 39 parallel zu den Stegen angeordnet. Die Stege 34, 35, 36 weisen runde Lochungen, Längsschlitze oder entspr. Ausnehmungen 40 auf, so daß ein Luftdurchtritt durch die Stegwandungen ermöglicht wird. Die Lochungen 40 sind vorzugsweise im oberen Bereich der Stegwandungen ausgebildet. Die Stege selbst bestehen vorzugsweise aus Aluminiumprofil.

## Patentansprüche

1. Bräunungsvorrichtung mit einem quer zur Längsachse der Bräunungsvorrichtung angeordneten, UV-Lampen aufweisenden Unterteil, das den Benutzer aufnimmt, einem UV-Lampen aufweisenden Oberteil, das den Benutzer von oben umgibt, und einer Vorrichtung zum Höhenbewegen des Oberteiles relativ zum Unterteil, wobei das Unterteil die Form eines Liegesessels hat und Oberteil und Unterteil einander entsprechende Abschnitte aufweisen, so daß der Benutzer dazwischen Platz findet,
**dadurch gekennzeichnet**, daß
a) die UV-Lampen (26, 27) im Unterteil (1) und im Oberteil (8) sich quer zur Längsachse der Bräunungsvorrichtung und über deren gesamte Breite von einer Seite zur anderen durchgehend erstreckend und in Längsrichtung parallel zueinander mit minimalem Abstand voneinander angeordnet sind,
b) das an einem seitlichen Träger (14) befestigte Oberteil (8) vom Unterteil (1) um eine horizontale, in Längsrichtung der Bräunungsvorrichtung verlaufende Achse nach oben wegschwenkbar oder relativ zum Unterteil heb- und senkbar angeordnet ist, und
c) zumindest im Unterteil (1) quer zu dessen Längsachse und parallel zur Längsachse der einzelnen Röhren (39) verlaufende durchgehende Stege bzw. Trennwände (34, 35, 36) vorgesehen sind, die Lochungen (40) für das Durchströmen von Kühlluft aufweisen, und daß die Stege bzw. Trennwände (34, 35, 36) zur Aufnahme einer Auflageplatte (38) ausgebildet sind.

2. Bräunungsvorrichtung nach Anspruch 1, dadurch gekennzeichnet, daß der Abstand zwischen Oberteil (8) und Unter teil (1) der Körperform entsprechend angepaßt ist und beide Teile im Brust- und Beckenbereich einen etwas größeren Abstand und im Bereich vom Oberschenkel abwärts einen etwas geringeren Abstand, als dem mittleren Abstand entspricht, aufweisen.

3. Bräunungsvorrichtung nach Anspruch 2, dadurch gekennzeichnet, daß die beiden die Rückenlehne (3) bildenden Abschnitte (3', 3'') sich nach unten erweiternd (ca. 2 -6°), die beiden die Sitzfläche (2) bildenden Abschnitte (2', 2'') sich zum Fußteil verengend (ca. 5 -10°) und die beiden die Fußstütze (4) bildenden Abschnitte (4', 4'') sich nach unten verengend (ca. 2 -6°) ausgebildet sind.

4. Bräunungsvorrichtung nach einem der Ansprüche 1 - 3, dadurch gekennzeichnet, daß an einem Sockel (5) seitlich ein vertikaler Träger (14, 19) angeordnet ist, der über mindestens einen Tragarm (15, 20) den beweglichen Oberteil (8) der Bräunungsvorrichtung relativ zum Unterteil (1) der Bräunungsvorrichtung beweglich aufnimmt, wobei die Bewegung wahlweise schwenkbar um die Längsachse oder vertikal erfolgt, um ein Einsteigen zu ermöglichen, und daß der Tragarm (15) mit dem Träger (14) so verbunden ist, daß der bewegliche Oberteil (8) der Bräunungsvorrichtung um den Befestigungspunkt von Tragarm (15) und Träger (14) nach oben schwenkbar ausgebildet ist bzw. daß der Träger (19) eine Führungsvorrichtung aufweist, in der der mit dem beweglichen Oberteil (8) der Bräunungsanlage befestigte Tragarm (20) höhenbeweglich geführt ist.

5. Bräunungsvorrichtung nach einem der Ansprüche 1 - 4, dadurch gekennzeichnet, daß die Schwenk- bzw. Höhenbewegung des Tragarmes (15, 20) im Träger (14, 19) durch Motorantrieb steuerbar ist, und daß der Antrieb für die Schwenk- bzw. Höhenbewegung über Seilzug bzw. Flaschenzug erfolgt, bzw. daß die Höhenbewegung durch Zahnstangenantrieb erfolgt.

6. Bräunungsvorrichtung nach einem der Ansprüche 1 - 5, dadurch gekennzeichnet, daß die gesamte Bräunungsanlage auf einer Wippenvorrichtung (22, 24, 25) befestigt ist, die motorisch und wahlweise nach einem Steuerprogramm die Bräunungsvorrichtung verschwenkt.

7. Bräunungsvorrichtung nach Anspruch 6, dadurch gekennzeichnet, daß die Wippenvorrichtung (22, 24, 25) die Bräunungsvorrichung um eine Querachse (22) um bis zu ca. zehn Winkelgrade mit extrem geringer Frequenz verschwenkt, und daß die Verschwenkung z.B. durch einen Exzenterantrieb (25) erfolgt, der in einer den Sockel 85) aufnehmenden Grundplatte (23) angeordnet ist.

8. Bräunungsvorrichtung nach einem der Ansprüche 1 - 7, dadurch gekennzeichnet, daß der Oberteil (8) und/oder der Unterteil (1) einen Seitenflügel (29, 28) mit UV-Lampen (32, 31) aufweist, der gegen den Hauptteil (8, 1) geneigt ist, wobei der Neigungswinkel zwischen 30° und 90° beträgt.

## Claims

1. Tanning device with a bottom part provided with UV-lamps positioned transversely to the longitudinal axis of the tanning device and which is taken up by the user, a top part provided with UV-lamps which surrounds the user from above, as well as an apparatus for the vertical movement of the top part relative to the bottom part, the bottom part being shaped like a reclining seat and the top part and bottom part have corresponding portions, so that the user finds space between them, characterized in that
a) the UV-lamps (26,27) in the bottom part (1) and in the top part (8) extend transversely to the longitudinal axis of the tanning device and over its entire width from one side to the other and are longitudinally positioned in parallel, minimum spaced manner,
b) the top part (8) fixed to a lateral support (14) can be pivoted away upwards from the bottom part (1) about a horizontal axis in the longitudinal direction of the tanning device or is arranged in raisable and lowerable manner relative to the bottom part and
c) at least in the bottom part (1) and transversely to its longitudinal axis and parallel to the longitudinal axis of the individual tubes (36) there are through webs or partitions (34,35,36), which have holes (40) for the passage of cooling air, and that the webs or partitions (34,35, 36) are constructed for receiving a supporting plate (38).

2. Tanning device according to claim 1, characterized in that the spacing between the top part (8) and the bottom part (1) is adapted to the body shape and both parts in the chest and pelvic region have a somewhat larger spacing and in the thigh region downwards have a somewhat smaller spacing than the average spacing.

3. Tanning device according to claim 2, characterized in that the two portions (3',3'') forming the back (3) widen downwards (approximately 2-6°), the two portions (2',2'') forming the seat surface (2) narrow towards the foot part (approximately 5-10°) and the two portions (4',4'') forming the foot-rest (4) narrow downwards (approximately 2-6°).

4. Tanning device according to one of the claims 1 to 3, characterized in that a vertical support (14,19) is laterally arranged on a base (5) and by means of at least one support arm (15,20) movably receives the movable top part (8) of the tanning device relative to the bottom part (1) of the tanning device, the movement taking place, as desired, pivotably about the longitudinal axis or vertically in order to permit getting in, and that the support arm (15) is so connected to the support (14) that the movable top part (8) of the tanning device is upwardly pivotable about the fixing point of the support arm (15) and the support (14) or that the support (19) has a guiding device, in which is vertically movably guided the support arm (20) fixed to the movable top part (8) of the tanning device.

5. Tanning device according to one of the claims 1 to 4, characterized in that the pivoting or vertical movement of the support arm (15,20) in the support (14,19) is controllable by a motor drive and that the drive for the pivoting or vertical movement takes place by means of a cable line or pulley block respectively, or the vertical movement takes place by a rack and pinion drive.

6. Tanning device according to one of the claims 1 to 5, characterized in that the complete tanning device is fixed to a rocker mechanism (22,24,25), which pivots the tanning device in motor manner and, if desired, according to a control program.

7. Tanning device according to claim 6, characterized in that the rocker mechanism (22,24,25) pivots the tanning device about a transverse axis (22) by up to approximately ten radians with an extremely low frequency and that the pivoting e.g. takes place by an eccentric drive (25), which is located in a base plate (23) receiving the base (85).

8. Tanning device according to one of the claims 1 to 7, characterized in that the top part (8) and/or the bottom part (1) has side wings (29,28) with UV-lamps (32,31), which is inclined against the main part (8,1), the angle of inclination being between 30 and 90°.

## Revendications

1. Dispositif de bronzage avec une partie inférieure disposée transversalement à l'axe longitudinal du dispositif de bronzage, munie de lampes à rayons UV, qui reçoit l'utilisateur, une partie supérieure munie de lampes à rayons UV qui entoure l'utilisateur d'en haut, et un dispositif pour le déplacement en hauteur de la partie supérieure relativement à la partie inférieure, la partie inférieure ayant la forme d'un fauteuil-couchette et la partie supérieure et la partie inférieure comportant des portions ou segments qui se correspondent de sorte que l'utilisateur trouve de la place entre eux, caractérisé en ce que
a) les lampes à rayons UV (26, 27) sont diposées dans la partie inférieure (1) et dans la partie supérieure (8) en s'étendant transversalement à l'axe longitudinal du dispositif de bronzage et sur toute sa largeur d'un côté à l'autre de manière continue, et en direction longitudinale parallèlement l'une à l'autre avec une distance minimale l'une de l'autre,
b) la partie supérieure (8) fixée au support latéral (14) est pivotable autour d'un axe horizontal disposé en direction longitudinale du dispositif de bronzage avec éloignement vers le haut par rapport à la partie inférieure (1) ou peut être élevée ou abaissée relativement à la partie inférieure, et
c) au moins dans la partie inférieure (1) sont prévues, transversalement à son axe longitudinal et parallèlement à l'axe longitudinal des tubes individuels (39), des traverses ou des parois de séparation continues (34, 35, 36) qui comportent des perforations (40) pour le passage d'air de refroidissement et les traverse ou parois de séparation (34, 35, 36) sont agencées pour recueillir une plaque d'appui (38).

2. Dispositif de bronzage selon la revendication 1 caractérisé en ce que la distance entre la partie supérieure (8) et la partie inférieure (1) est adpatée de manière à correspondre à la forme du corps et les deux parties présentent dans la région de la poitrine et du bassin une distance un peu supérieure et dans la région allant de la cuisse vers le bas une distance un peu inférieure à celle qui correspond à la distance moyenne.

3. Dispositif de bronzage selon la revendication 2 caractérisé en ce que les deux portions (3', 3'') formant le dossier (3) sont agencées en s'écartant vers le bas (d'environ 2 à 6°), les deux portions (2', 2'') formant la surface de siège (2) sont agencées en se rapprochant (d'environ 5 à 10°) en direction des pieds, et les deux portions (4', 4'') formant le support des pieds (4) sont agencées en se rapprochant (d'environ 2 à 6°) vers le bas.

4. Dispositif de bronzage selon l'une des revendications 1 à 3 caractérisé en ce qu'un support vertical (14, 19) est disposé latéralement à un socle (5), support qui reçoit au moyen d'au moins un bras de support (15, 20) la partie supérieure mobile (8) du dispositif de bronzage de manière à permettre son déplacement relativement à la partie inférieure (1) du dispositif de bronzage, le déplacement ayant lieu en option par pivotement autour de l'axe longitudinal ou verticalement pour permettre l'entrée et le bras de support (15) est assemblé au support (14) de manière telle que la partie supérieure mobile (8) du dispositif de bronzage soit agencée de façon pivotable vers le haut autour du point de fixation du bras de support (15) et du support (14) ou que le support (19) comporte un dispositif de guidage dans lequel est guidé de façon déplaçable en hauteur le bras de support (20) fixé à la partie supérieure mobile (8) de l'installation de bronzage.

5. Dispositif de bronzage selon l'une des revendications 1 à 4 caractérisé en ce que le pivotement ou le déplacement en hauteur du bras de support (15, 20) dans le support (14, 19) peut être commandé par un moteur et que l'entraînement pour le mouvement de pivotement ou le déplacement en hauteur a lieu par commande par câble ou par palan ou que le déplacement en hauteur a lieu par crémaillère et pignon.

6. Dispositif de bronzage selon l'une des revendications 1 à 5 caractérisé en ce que toute l'installation de bronzage est fixée sur un dispositif de basculement (22, 24, 25) qui fait pivoter le dispositif de bronzage au moyen d'un moteur et en option selon un programme de commande.

7. Dispositif de bronzage selon la revendication 6 caractérisé en ce que le dispositif de basculement (22, 24, 25) fait pivoter de jusqu'à environ 10° avec une fréquence extrêmement faible le dispositif de bronzage autour d'un axe transversal (22) et le pivotement a lieu par exemple au moyen d'un mécanisme à excentrique (25) qui est disposé dans une plaque de base (23) recevant le socle (5).

8. Dispositif de bronzage selon les revendications 1 à 7 caractérisé en ce que la partie supérieure (8) et/ou la partie inférieure (1) sont munies d'une aile latérale (29, 28) à lampes à rayons UV, aile qui est inclinée par rapport à la partie principale (8, 1), l'angle d'inclinaison étant compris entre 30° et 90°.
